# EUROPEAN PATENT SPECIFICATION

(11) **EP 0 341 988 B1**
(45) Date of publication and mention of the grant of the patent: **13.01.1993**
(21) Application number: 89304699.5
(22) Date of filing: 09.05.1989
(51) Int. Cl.: A61M 25/00, A61M 29/02

(54) **Prostate balloon dilator**
Prostata-Ballondilator
Dilateur prostatique à ballon

(30) Priority: 10.05.1988 US 192432
(43) Date of publication of application: 15.11.1989
(73) Proprietor: American Medical Systems, Inc., Minnetonka Minnesota 55343 (US)
(72) Inventor: Reddy, Pratap Konudulla, Bloomington State of Minnesota (US); Mikulich, Michael Andrew, Shakopee State of Minnesota (US); Clark, David Winston, New Hope State of Minnesota (US)
(74) Representative: Bradbrook, Geoffrey William

(56) References cited:
- DE-A- 2 433 959
- DE-A- 3 425 437
- US-A- 2 799 273
- US-A- 3 509 884
- US-A- 4 651 721
- US-A- 4 660 560
- US-A- 4 702 252

## Description

The invention relates to a catheter having inflatable means for dilation of the prostate urethra, according to the first part of claim 1. Such a catheter is disclosed by US-A-2 799 273.

Balloon catheters are widely used for dilation of undesirable tissue in body vessels. For instance, U.S. Patent No. 4,636,195 discloses removal of constrictions caused by deposition of plaque in arteries by a balloon catheter. The disclosed catheter has two smaller ring balloons spaced around a central dilation balloon. The smaller ring balloons provide a chamber around a body of plaque, and help to hold the catheter body in place. Other multiple balloon catheters are disclosed in U.S. Patents 4,573,966 and 4,610,662. The two balloons of these catheters are placed in a vascular passageway to seal off a constricted area, and dissolving fluid is supplied to the enclosed area to dissolve the constrictive tissue.

Treatment of obstructive tissue in the prostate urethra with a balloon catheter is disclosed in U.S. Patent 4,660,560 which describes a catheter having a Foley balloon for anchoring the catheter by inflation within the bladder, and an annular balloon for dilation of the prostate urethra. Proper location of the annular balloon is attained by introducing a cystoscope into the prostate urethra. The operation of the cystoscope to determine the location of the prostate urethra with respect to the bladder neck is cumbersome, and is avoided by the invention as described below.

A US-A-2799273 describes a haemostatic catheter comprising a pair of balloons positioned so that one balloon may be located in the bladder and the other, pressing against the first balloon when inflated, within the prostate. DE-A-2433959 describes a rectal catheter comprising a pair of balloons for rectal surgery. US-A-3509884 describes a rectal catheter having an inflatable balloon and stop means for controlling the distance of penetration of the catheter.

The solution to the problem posed by the application is given by the features in the characterising portion of claim 1.

As used herein, the term impervious means both that the material from which the catheter is made is impervious and also that the catheter tube is impervious in that the tubular wall does not have any openings along its length. The terms "distal" and "proximal" are used relative to the patient and not the operator.

In a preferred embodiment of the invention, the dilation means is a dilation balloon and the location means is a location balloon of lesser size than the dilation balloon.

The dilation balloon is preferably made of a limited distensible material such that the balloon can not expand from its initial deflation diameter to substantially beyond a predetermined diameter regardless of the internal pressure applied to the balloon.

The apparatus is intended for a method for the treatment of benign prostatic hyperplasia.

To facilitate the placement of the catheter, the location balloon is axially mounted on said catheter at a distance from said dilation balloon such that the location balloon is positioned at the bulbous urethra distal from the external sphincter to hold the catheter in place when the dilation balloon is positioned at the prostate urethra, and said location balloon on inflation is sized to fit in the bulbous urethra.

In a further embodiment the distal end of the dilation balloon is provided with an integral protuberance which makes it slightly stiffer and thicker than the remainder of the balloon whereby palpation is enhanced when the protuberance is placed at the apex of the prostate. This embodiment facilitates correct placement of the dilation balloon in the prostate urethra and the location balloon in the bulbous urethra.

A preferred embodiment of the invention is illustrated in the accompanying drawings, in which:-

FIG. 1 illustrates a dual balloon catheter according to the invention as it is inserted in the male urethra, showing the balloons in inflated condition.

FIG. 2 is a fragmentary sectional view of the device of FIG. 1.

FIG. 3 is a cross-sectional view of the device of FIG. 1 showing the balloons in inflated condition.

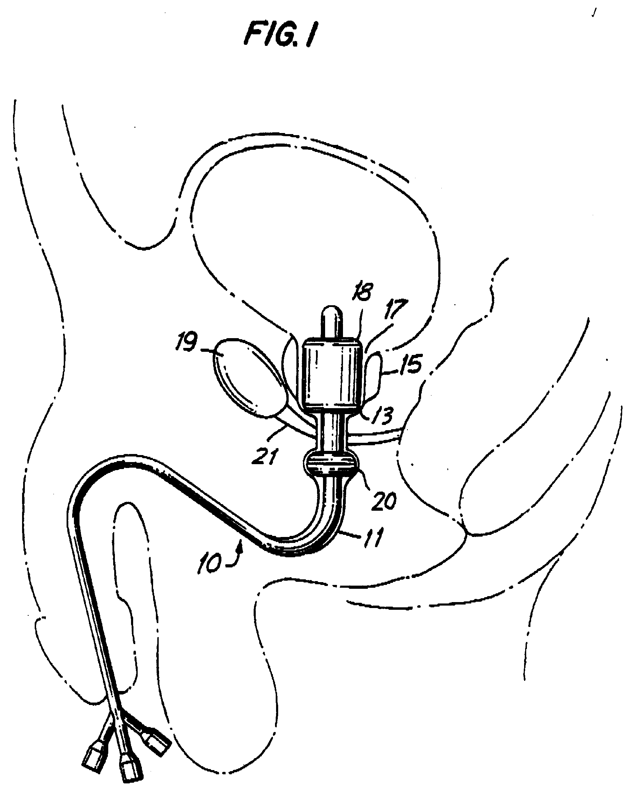

The embodiment illustrated in FIG. 1 comprises a dual balloon catheter 10 positioned within the male urethra 11. Inflatable location balloon 20 located at the bulbous urethra anchors the device 10 in place and secures it against significant movement in the longitudinal directions, particularly in the direction of the bladder. Inflatable dilation balloon 18 is located at the prostate urethra 13 near the prostate 15 and extends into the bladder neck 17. FIG. 1 shows the location of the male urethra relative to the pubic bone 19 and the urogenital diaphragm (or pelvic floor) 21.

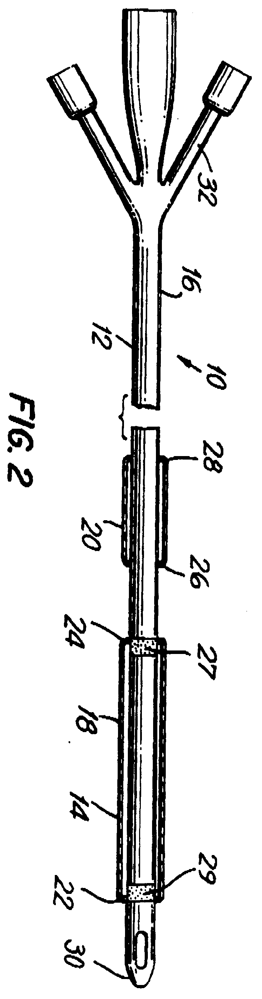

FIG. 2 shows the device 10 comprising catheter tube 12 having proximal section 14 for location toward the center of the body on proper insertion of the device 10 in the body, and distal section 16 for location away from the center of the body on proper insertion of the device 10 in the body.

The catheter tube 12 is formed of a material which is flexible enough to follow the bends in the urethra on insertion of the device 10. The material should be rigid enough, however, to allow the device 10 to pass over any obstructions within the urethra on insertion of the device 10. The catheter tube is composed of or covered by a biocompatible material to avoid irritations and complications in the urethra. Suitable biocompatible materials which can be made into tubing of suitable flexibility and rigidity are silicone, polyester, polyvinylchloride, and polyurethane.

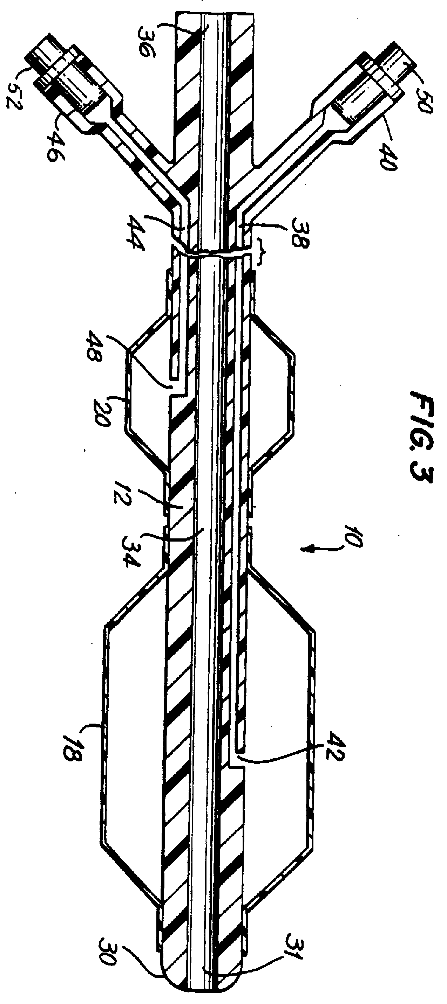

Mounted on the proximal section 14 is axial dilation balloon 18 and axial location balloon 20. Dilation balloon 18 is in communication with the interior of catheter tube 12, as described in more detail with reference to FIG. 3. Dilation balloon 18 may be replaced by multiple balloons, although one dilation balloon is preferred. The location balloon 20 is at a distance from dilation balloon 18 such that location balloon 20 is at the bulbous urethra when the dilation balloon 18 is at the prostate urethra.

Balloons 18 and 20 are formed of a biocompatible material such as silicone elastomer, polyvinylchloride, polyester, natural or synthetic rubber, or polyurethane. Dilation balloon 18 in general is made such that it does not expand beyond a predetermined diameter. Such limited expansion of the balloon 18 prevents overdilation and thus prevents damage to the prostate urethra during dilation. In one embodiment, the limited expansion is attained by making the dilation balloon of a non-distensible material such as polyvinylchloride, polyethylene terephthalate, or polyethylene. In the deflated state, such non-distensible balloon is in a folded configuration. Such limited expansion, alternatively, may be attained by making the balloon 18 of a limited distensible composite material as defined above. Multi-layer limited distensible materials of use in the present invention are described in U.S. Patent 4,637,396 with respect to dilation "balloon 12" having a three layer wall. The inner layer is an elastic impervious polyurethane membrance, the middle layer is a knitted fabric tube, and the outer layer is an elastic impervious polyurethane or silicone membrance. An elastomeric outer layer or cover made of silicone is particularly preferred because it increases patient comfort by providing a smooth outer surface for the deflated balloon. Other multi-layer limited distensible materials of use in the present invention are the bistable materials described in U.S. Patent 4,651,721, the disclosure of which is incorporated by reference. The limited distensible material may be part of a three layer composite having an outer layer which is elastic and may be formed of silicone elastomer, a middle layer of a bistable material such as polyester/polyurethane fabric, and an inner layer which is elastic and impervious and may be formed of silicone elastomer. The bistable material may be a fabric made of yarns composed of non-distensible fibers such as polyester fibers and distensible fibers such as polyurethane fibers. The distensible fibers allow for collapse of the balloon to about the outer diameter of the catheter tube 12, and the non-distensible fibers allow for inflation of the balloon to the predetermined diameter. If desired a suitable lubricant, for example silicone oil, may be placed between the layers, particularly the outer smooth silicone layer and its adjacent inner layer, to ensure smooth relative sliding motion of each layer with respect to the adjacent layer.

The predetermined diameter of the dilation balloon 18 is generally about 25 mm to about 40 mm.

The dilation balloon 18 has a proximal end 22 and a distal end 24, and the location balloon 20 has a proximal end 26 and a distal end 28. The distance between the distal end 24 of the dilation balloon 18 and the proximal end 26 of the location balloon 20 varies with the relative location of the prostate urethra and the bulbous urethra in a patient, and is usually from about 1 cm to about 4 cm, most typically 1 cm. The device 10 has a proximal end 30 and a distal end 32. The proximal end 22 of the dilation balloon 18 is usually about 1 cm to about 3 cm from the proximal end 30 of the device 10. Optionally, the dilation balloon extends beyond the proximal end 30.

The length of the dilation balloon 18, that is the distance between the proximal end 22 and the distal end 24, depends upon the size of the prostate urethra of a patient. In general, such length of balloon 18 is about 3 cm to about 6 cm, commonly 4 cm, in accordance with the general size of a male prostate urethra. The dilation balloon 18 may extend beyond the prostate urethra into the bladder neck, so allowing for some flexibility in use of a limited number of lengths of dilation balloons for different lengths of prostate urethras.

The length of the location balloon 20, that is the distance between the proximal end 26 and the distal end 28, is such that the balloon fits in the bulbous urethra of a patient. The balloon 20 need not occupy the entire space of the bulbous urethra. The functions of the balloon 20 are locating the prostate urethra, and fixating the device 10 against movement within the urethra, particularly against movement in the direction of the bladder. As a general rule, the length of the location balloon 20 is about 1 cm to 6 cm.

Radiopaque markers 27 and 29 are conveniently used to guide fluoroscopy examination.

FIG. 3 shows one embodiment of the dilation balloon 18, as attached to catheter tube 12. Drainage lumen 34 extends from the opening 31 at proximal end 30 through the catheter tube 12 to bladder drainage hole 36. The drainage lumen is an optional feature of device 10. A dilation lumen 38 communicates between a dilation balloon fill port 40 and the dilation balloon 18 through a dilation balloon opening 42. An inflation lumen 44 communicates between a location balloon fill port 46 and the location balloon 20 through a location balloon opening 48. Each of balloon fill ports 40 and 46 have a catheter valve or syringe fitting 50 and 52, respectively, to allow for connection of the fill ports with syringes for injection of fluid.

The invention will be more particularly described with reference to the following Examples which illustrate the method according to the invention.

### EXAMPLE 1

Before insertion into the urethra, device 10 is in a completely deflated state such that balloons 18 and 20 are collapsed against the exterior of catheter tube 12.

The device 10 is guided into and along the urethra. To facilitate insertion, the device 10 may be guided over a previously inserted guide wire or the drainage lumen 34 may contain a guide wire so increasing the stiffness of the device 10.

The location balloon 20 is properly located at the bulbous urethra. The doctor feels the bulbous urethra by rectal palpation or determines the position of the bulbous urethra by other means such as fluoroscopic guidance. Conveniently, the location balloon 20 is inflated to at least some extent when it is in the vicinity of the bulbous urethra as determined by the doctor by rectal palpation or other known means. The location balloon 20 is inflated to its full extent after location at the bulbous urethra.

For inflation of the balloon 20, the inflation lumen 44 leading to the location balloon 20 is connected to an inflating means such as a syringe, and inflation fluid is injected to inflate balloon 20 by fluid pressure. The inflation fluid is preferably a radiopaque fluid for post insertion X-ray examination and viewing of the device while in the body. Hypaque-25 Registered Trade Mark and Renografin-60 Registered Trade Mark are examples of suitable radiopaque fluids. Sufficient fluid is injected to secure the device 10 in its proper position. Once the inflated location balloon 20 is properly located at the bulbous urethra distal from the external sphincter, the doctor knows that the dilation balloon 18 is at its proper location at the prostate urethra since the location balloon 20 of device 10 is at a distance from the dilation balloon 18 such that when location balloon 20 is at the bulbous urethra then the dilation balloon 18 is at the prostate urethra. In this manner, dilation of the external sphincter, and possible harmful effects of such dilation, is avoided.

The dilation balloon 18 is now connected to a pressure gauge through the dilation lumen 38, and inflated. The proper inflation pressure for maximum dilation is attained by injecting a volume of fluid equal to the predetermined capacity of balloon 18, or by determining the maximum pressure on the pressure gauge when exerting pressure on the balloon 18. As is known in the dilation art in general, fluoroscopy aids in visualizing the extent of the balloon's expansion and in monitoring the dilation procedure.

In the preferred embodiment when the dilation balloon 18 is made of limited distensible material as described above, the balloon 18 expands to its predetermined maximum diameter.

After dilation, balloons 18 and 20 are deflated, and the device 10 is removed. Removal is facilitated with minimal distress to the patient when the dilation balloon is made of a multi-layer material, wherein the outer layer is silicone and the inner layer is, for example, a polyester. In such an embodiment the outer layer when deflated, because of its smooth and elastic character, bends over and folds in any wings and spikes in the deflated inner layer to provide a smooth outer surface of acceptable small diameter for easy withdrawal.

### EXAMPLE 2

As on alternative to the above procedure, placement of the device according to the invention may be performed by a modification of the palpation technique described in Example 1. This alternative procedure comprises the following steps:-

After placing the patient in a lithotomy position, a Council catheter is passed into the bladder. If the bladder is empty, it is filled with approximately 100 ml. of sterile water or normal saline. A guide wire is passed through the Council Registered Trade Mark catheter into the bladder and the catheter is then removed.

A device according to the invention is passed over the guide wire until both the dilation balloon and location balloon are in the bladder.

A filled 3 ml. syringe is attached to a two-way stopcock and this assembly is connected to the location balloon port 46. With a finger in the patient's rectum, the doctor palpates the apex of the prostate. The location balloon is inflated with approximately 1 ml. of contrast solution and the stopcock valve is closed.

The device is gently pulled until the location balloon is palpated at the apex of the prostate. If there is any resistance to this movement, the stopcock valve is opened and the location balloon is gradually deflated until it slides through to the apex.

The device is then withdrawn an additional 1.5 to 2 cm. until the distal end 24 of the dilation balloon is palpated at the apex of the prostate. To facilitate this operation the wall of the balloon adjacent said distal end is made slightly thicker and stiffer than the remainder of the dilation balloon by providing an integral protuberance (not shown) which may be made from the same material as the outer wall of the dilation balloon or from a different material attached to said wall without disturbing the general smoothness of the outermost surface of the balloon.

Since the rectum is closer to the prostate urethra than it is to the bulbous urethra, palpation of the protuberance at the base of the dilation balloon when it is at the apex of the prostate should be easier than palpation of the location balloon in the bulbous urethra. However, it is to be understood that when the said protuberance is palpated at the apex of the prostate, the location balloon is in the bulbous urethra below the external sphincter. The location balloon is now inflated until resistance is met (approximately 1.3 ml. total volume) and the stopcock valve is then closed.

Dilation of the prostate is performed as in Example 1 and rectal palpation will confirm proper positioning during dilation.

After prostate dilation, both balloons are deflated and the device is removed as previously described.

## Claims

1. An apparatus for dilation of the prostate urethra comprising:
an impervious urinary catheter (10) for insertion in the prostate urethra (11), said catheter having a proximal section (14) and a distal section (16);
dilation means (18) mounted on said proximal section (14) of the catheter;
location means (20) mounted on the catheter;
and activation means located at said distal section of the catheter for dilating said dilation means (18),
characterised in that the location means (20) are mounted on the catheter at a distance of at least about 1 cm from the dilation means so that the location means is positionable at the bulbous urethra distal from the external sphincter and fixable thereat to hold the catheter in place when the dilation means is positioned at the prostate urethra, and activation means are provided to fix said location means.

2. An apparatus according to claim 1 wherein said dilation means is a dilation balloon (18) and said location means is a location balloon (20) of lesser size than the dilation balloon.

3. An apparatus according to claim 2 wherein said dilation balloon is made of a multi-layer limited distensible material.

4. An apparatus according to claim 3 wherein said multi-layer limited distensible material comprises an inner and outer layer of silicone and a middle layer of a limited distensible fabric, or an outer layer of silicone and an inner layer of polyester.

5. An apparatus according to claim 2 wherein said dilation balloon 18 and said location balloon (20) each have a proximal end and a distal end, and the distal end (24) of the dilation balloon has an integral protuberance of thicker and stiffer material than the remainder of the balloon.

6. An apparatus according to any one of claims 3 to 5 wherein a lubricant is placed between the layers of the multi-layered material.

7. An apparatus according to any one of claims 2 to 6 wherein said catheter has three lumens extending from said proximal section, one lumen (38) in communication with said dilation balloon, one lumen (44) in communication with said location balloon, and one lumen (34) adapted for communication with the bladder of a subject.

## Patentansprüche

1. Vorrichtung für die Dilatation der Prostata-Urethra, mit: einem undurchlässigen Urin-Katheter (10) zum Einsetzen in die Prostata-Urethra (11), wobei dieser Katheter einen proximalen Abschnitt (14) und einen distalen Abschnitt (16) aufweist; eine Dilatationseinrichtung (18), die am proximalen Abschnitt (14) des Katheters montiert ist;
eine Positionierungseinrichtung (20), die am Katheter montiert ist;
und eine Aktivierungseinrichtung, die am distalen Abschnitt des Katheters montiert ist, zum Ausdehnen der Dilatationseinrichtung (18);
dadurch gekennzeichnet, daß die Positionierungseinrichtung (20) am Katheter in einem Abstand von wenigstens etwa 1 cm von der Dilatationseinrichtung montiert ist, so daß die Positionierungseinrichtung an der Bulbus-Urethra distal vom äußeren Schließmuskel positionierbar und dort fixierbar ist, um den Katheter an Ort und Stelle zu halten, wenn die Dilatationseinrichtung an der Prostata-Urethra angeordnet ist, und daß eine Aktivierungseinrichtung vorhanden ist, um die Positionierungseinrichtung zu fixieren.

2. Vorrichtung nach Anspruch 1, bei der die Dilatationseinrichtung ein Dilatationsballon (18) und die Positionierungseinrichtung ein Positionierungsballon (20) von geringerer Größe als der Dilatationsballon ist.

3. Vorrichtung nach Anspruch 2, bei der der Dilatationsballon aus einem mehrschichtigen begrenzt dehnbaren Material besteht.

4. Vorrichtung nach Anspruch 3, bei der das mehrschichtige begrenzt dehnbare Material eine innere Schicht und eine äußere Schicht aus Silikon sowie eine mittlere Schicht aus einem begrenzt dehnbaren Gewebe, oder eine äußere Schicht aus Silikon und eine innere Schicht aus Polyester umfaßt.

5. Vorrichtung nach Anspruch 2, bei der der Dilatationsballon (18) und der Positionierungsballon (20) jeweils ein proximales Ende und ein distales Ende aufweisen und das distale Ende (24) des Dilatationsballons einen integralen Vorsprung aus dickerem und steiferem Material als dem Rest des Ballon hat.

6. Vorrichtung nach einem der Ansprüche 3 bis 5, bei der ein Gleitmittel zwischen den Schichten des mehrschichtigen Materials angeordnet ist.

7. Vorrichtung nach einem der Ansprüche 2 bis 6, bei der der Katheter drei Durchgangskanäle aufweist, die sich vom proximalen Abschnitt weg erstrecken, wobei ein Durchgangskanal (38) in Kommunikation mit dem Dilatationsballon steht, ein weiterer Durchgangskanal (44) in Kommunikation mit dem Positionierungsballon steht und ein Durchgangskanal (34) für die Kommunikation mit der Blase einer Person ausgestattet ist.

## Revendications

1. Appareil pour la dilatation de l'urètre prostatique, comportant :
un cathéter urinaire étanche (10) à insérer dans l'urètre prostatique (11), ledit cathéter ayant un tronçon proximal (14) et un tronçon distal (16) ;
un moyen de dilatation (18) monté sur ledit tronçon proximal (14) du cathéter ;
un moyen de positionnement (20) monté sur le cathéter ;
et un moyen d'activation placé audit tronçon distal du cathéter pour dilater ledit moyen de dilatation (18),
caractérisé en ce que le moyen de positionnement (20) est monté sur le cathéter à une distance d'au moins environ 1 cm du moyen de dilatation afin que le moyen de positionnement puisse être placé à l'urètre membraneux, du côté distal par rapport au sphincter externe, et qu'il puisse y être fixé pour maintenir le cathéter en place lorsque le moyen de dilatation est positionné à l'urètre prostatique, et des moyens d'activation sont prévus pour fixer ledit moyen de positionnement.

2. Appareil selon la revendication 1, dans lequel ledit moyen de dilatation est un ballonnet (18) de dilatation et ledit moyen de positionnement est un ballonnet (20) de positionnement d'une dimension inférieure à celle du ballonnet de dilatation.

3. Appareil selon la revendication 2, dans lequel ledit ballonnet de dilatation est réalisé en une matière multicouche pouvant être distendue de façon limitée.

4. Appareil selon la revendication 3, dans lequel ladite matière multicouche pouvant être distendue de façon limitée comprend des couches intérieure et extérieure en silicone et une couche médiane en une étoffe pouvant être distendue de façon limitée, une couche extérieure en silicone et une couche intérieure en polyester.

5. Appareil selon la revendication 2, dans lequel ledit ballonnet (18) de dilatation et ledit ballonnet (20) de positionnement ont chacun une extrémité proximale et une extrémité distale, et l'extrémité distale (24) du ballonnet de dilatation est réalisée d'une seule pièce avec une protubérance en matière plus épaisse et plus rigide que celle de la partie restante du ballonnet.

6. Appareil selon l'une quelconque des revendications 3 à 5, dans lequel un lubrifiant est placé entre les couches de la matière multicouche.

7. Appareil selon l'une quelconque des revendications 2 à 6, dans lequel ledit cathéter présente trois lumières s'étendant depuis ledit tronçon proximal, une lumière (38) de communication avec ledit ballonnet de dilatation, une lumière (44) en communication avec ledit ballonnet de positionnement et une lumière (34) destinée à communiquer avec la vessie d'un sujet.
